**Europäisches Patentamt**

(19) **European Patent Office**

**Office européen des brevets**

(11) Numéro de publication: **0 000 110**
**B1**

(12) **FASCICULE DE BREVET EUROPEEN**

(45) Date de publication du fascicule du brevet: **29.08.84**

(21) Numéro de dépôt: **78400011.9**

(22) Date de dépôt: **05.06.78**

(51) Int. Cl.³: **C 07 C 57/04,** C 07 C 51/00, C 07 C 51/44, C 07 C 51/42

(54) **Procédé d'obtention d'acide acrylique à partir de ses solutions dans le phosphate de tri-n-butyle.**

(30) Priorité: **14.06.77 FR 7718136**

(43) Date de publication de la demande:
**20.12.78 Bulletin 78/01**

(45) Mention de la délivrance du brevet:
**29.08.84 Bulletin 84/35**

(84) Etats contractants désignés:
**BE CH DE FR GB NL SE**

(56) Documents cités:
**FR-A-1 558 432**

(73) Titulaire: **RHONE-POULENC CHIMIE DE BASE
25, quai Paul Doumer
F-92408 Courbevoie (FR)**

(72) Inventeur: **Biola, Georges
30, rue Montferrad
F-69500 Bron (FR)**
Inventeur: **Komorn, Yves
19, Avenue Joannes Hubert
69160-Tassin La Demi Lune (FR)**
Inventeur: **Schneider, Gérard
7, avenue de la Prévoyance
F-69300 Caluire (FR)**

(74) Mandataire: **Dutruc-Rosset, Marie-Claude
RHONE POULENC RECHERCHES Service
Brevets Chimie et Polymères 25, Quai Paul
Doumer
F-92408 Courbevoie Cedex (FR)**

Courier Press, Leamington Spa, England.

## Description

La présente invention a pour objet un procédé d'obtention d'acide acrylique pur à partir de ses solutions dans le phosphate de tri-n-butyle, par une technique particulière de distillation.

On a proposé selon une technique de l'art antérieur décrite dans le brevet américain 3 644 179 de séparer en continu un mélange de composés dont certains sont sensibles à la chaleur à l'aide d'un évaporateur à film mince.

On sait que la séparation de l'acide acrylique à partir de ses milieux réactionnels de fabrication, par exemple à partir du produit brut d'oxydation de propylène et/ou de l'acroléine, pose une problème délicat et de nombreux procédés ont été proposés à cet effet. On connait notamment une technique d'absorption en phase gazeuse ou d'extraction en phase liquide de l'acide acrylique au moyen de divers solvants. L'acide acrylique est ensuite isolé par distillation ou directement estérifié dans le milieu d'extraction (brevet français 1 558 432 du 14 Décembre 1967 — brevet français 1 452 566 du 5 Novembre 1965).

Parmi les solvants déjà préconisés, le phosphate de tri-n-butyle est particulièrement efficace. Mais la séparation de l'acide acrylique à partir de ses solutions dans ce solvant se heurte à de nombreuses difficultés. En effet, le phosphate de tributyle se dégrade lorsqu'il est soumis à des températures élevées, spécialement en présence d'acides et/ou d'eau, de sorte que l'épuisement en acide acrylique de la solution, par distillation à la pression normale ou sous une pression légèrement réduite se traduit par une perte non négligeable de solvant. D'autre part, pour obtenir un bon rendement d'absorption ou d'extraction de l'acide acrylique à l'aide du phosphate de tributyle recyclé après distillation, il est nécessaire que celui-ci ait un titre aussi faible que possible en acide acrylique. Pour éviter la dégradation du solvant et abaisser le plus possible la teneur de celui-ci en acide acrylique, on pourrait envisager d'effectuer la distillation sous vide poussé. Mais, dans ce cas, on rencontre des difficultés de récupération de l'acide acrylique en raison de son point de cristallisation relativement élevé (13,5°C) correspondant à une pression de vapeur de quelques mm de mercure.

Il a maintenant été trouvé un procédé qui remédie aux inconvénients précités dans la mesure où il permet de séparer sans difficultés l'acide acrylique, de recueillir le phosphate de tri-n-butyle sensiblement exempt de cet acide et d'éviter la dégradation du phosphate. D'autres avantages de ce procédé apparaitront dans la description qui va suivre.

La présente invention a donc pour objet un procédé d'obtention d'acide acrylique pur consistant à absorber le gaz réactionnel issu de l'oxydation du propylène et/ou de l'acroléine par un agent d'absorption et à séparer de cette solution d'une part l'acide acrylique pur et d'autre part l'agent d'absorption à un degré de pureté suffisante pour son recyclage, caractérisé en ce que:

l'agent d'absorption est le phosphate de tri-n-butyle et la séparation du phosphate de tri-n-butyle et de l'acide acrylique est réalisée par une combinaison de traitements thermiques évitant à la fois la décomposition du phosphate de tri-n-butyle et la cristallisation de l'acide acrylique dans les appareils, ladite combinaison consistant:

1 — à créer dans un premier appareil une phase vapeur comprenant sensiblement tout l'acide acrylique et une partie du phosphate de tri-n-butyle par chauffage de la solution entre 110 et 150°C sous 1 à 30 mm de mercure et avec un temps de séjour inférieur à 30 minutes de manière à éviter la dégradation du phosphate de tri-n-butyle.

2 — à traiter en continu les 2 phases ainsi créées comme suit:

d'une part, recycler comme agent d'absorption le phosphate de tri-n-butyle concentré dans la phase liquide et d'autre part, évacuer le mélange gazeux d'acide acrylique et de phosphate de tri-n-butyle vers une unité de distillation où le mélange éventuellement condensé en tout ou en partie est traité de manière à ce que le produit en bas de colonne contienne de 1 à 20 % d'acide acrylique, à ce que la température en pied de colonne se situe entre 120 et 150°C sous 5 à 30 mm de mercure et que le temps de séjour ne soit pas supérieur à 1 heure de telle façon que la dégradation du phosphate de tri-n-butyle et la cristallisation de l'acide acrylique en tête de colonne soit évitée.

La solution de départ peut être d'origine quelconque. Elle peut résulter par exemple de l'absorption de l'acide acrylique par le phosphate de tri-n-butyle à partir de mélange gazeux obtenu par oxydation du propylène et/ou de l'acroléine, comme il est décrit dans le brevet français 1 558 432 déjà cité. Elle peut également être issue de l'extraction liquide-liquide de solutions aqueuses d'acide acrylique par le phosphate de tri-n-butyle. Elle peut en outre contenir sans inconvénient de faibles quantités d'autres acides organiques plus légers que l'acide acrylique, en particulier d'acide acétique, formé(s) en même temps que l'acide acrylique et entrainé(s) par le solvant. Comme ou le verra plus loin, ces acides peuvent aisément être isolés dans un stade complémentaire, dans le cadre du procédé de l'invention. Par ailleurs, si au cours des opérations d'absorption ou d'extraction de l'acide acrylique par le phosphate de tributyle, de l'acroléine et/ou de l'eau ont été entrainées, il est avantageux de débarrasser la solution de ces composés, préalablement à la mise en oeuvre du procédé selon l'invention, par exemple par chauffage, éventuellement avec injection d'un gaz.

ces acides peuvent aisément être isolés dans un stade complémentaire, dans le cadre du procédé de l'invention. Par ailleurs, si au cours des opérations d'absorption ou d'extraction de l'acide acrylique par le phosphate de tributyle, de l'acroléine et/ou de l'eau ont été entraînées, il est avantageux de débarrasser la solution de ces composés, préalablement à la mise en oeuvre du procédé selon l'invention, par exemple par chauffage, éventuellement avec injection d'un gaz.

La concentration en acide acrylique de la solution de départ est indifférente, le procédé de l'invention s'appliquant aussi bien et présentant autant d'intérêt dans le cas des solutions concentrées ou diluées d'acide acrylique. A titre purement indicatif, on peut dire que la solution initiale peut renfermer de 5 à 50 % en poids d'acide acrylique.

Suivant le premier stade opératoire de la technique selon l'invention, on constitue une phase vapeur comprenant sensiblement tout l'acide acrylique et une partie du phosphate de tri-n-butyle, par chauffage du liquide initial dans des conditions qui évitent la dégradation du phosphate de tributyle. A cet égard la limite supérieure de température constitue un facteur essentiel. Il convient de ne pas dépasser notablement 160°C, la gamme de températures la plus avantageuse se situant entre 110°C et 150°C. La durée du chauffage a également une certaine importance et doit être contrôlée; les temps de séjour sont d'autant plus courts que la température adoptée est plus élevée; pour la gamme de températures précitée, des temps de séjour compris entre environ 30 minutes et 5 secondes conviennent généralement pour l'obtention d'une phase vapeur contenant, selon l'invention, la quasi-totalité de l'acide acrylique et une fraction du phosphate de tributyle sans dégradation du solvant en phase liquide ou vapeur.

La pression à appliquer se déduit des autres conditions opératoires; sa détermination est donc à la portée de l'homme de l'art. A titre indicatif, on peut dire que l'on travaile de préférence à une pression inférieure à la pression atmosphérique, dans une gamme se situant généralement entre 1 et 30 mm de mercure.

En pratique, pour la réalisation du premier stade opératoire, qui peut comprendre un ou plusieurs étages, on a recours aux divers moyens de type connu permettant une vaporisation dans les conditions qui viennent d'être décrites: par exemple, on peut effectuer une série de vaporisations instantanées en continu, ou utiliser une colonne à plateaux chauffants, ou mieux appliquer la technique bien connue du film mince (voir par exemple M. Loncin "Les opérations unitaires de génie chimique" pp. 408—411), ou encore combiner ces diverses techniques.

Le liquide restant après cette phase de vaporisation est constitué par du phosphate de tri-n-butyle sensiblement exempt d'acide acrylique et de tout autre acide organique léger qui aurait pu être présent dans le liquide de départ. L'obtention de phosphate de tri-n butyle épuisé en acide organique, après ce premier stade opératoire, représente un des importants avantages du procédé selon l'invention. En effet, grâce à cette récupération rapide d'une fraction importante du solvant, celle-ci est soumise pendant une période minimale à un effet thermique et les meilleures conditions sont remplies pour éviter la dégradation du phosphate; de plus, comme le solvant récupéré n'est pas chargé en acide, il peut être recyclé directement aux opérations d'extraction ou d'absorption de l'acide acrylique à partir de solutions ou gaz en contenant, où il donne son rendement maximum d'extraction ou d'absorption.

Suivant le deuxième stade opératoire caractéristique du procédé de l'invention, on soumet la phase vapeur formée dans le premier stade, après condensation partielle ou totale, à une distillation permettant de recueillir de l'acide acrylique de haute pureté. Les conditions de température de cette distillation sont critiques: en effet, le point d'ébullition du liquide ne doit pas dépasser la température à laquelle le phosphate de tributyle se dégrade sensiblement, c'est-à-dire environ 160°C, la gamme de travail la plus avantageuse se situant entre 120 et 150°C. Ce résultat ne peut pas être atteint de manière classique par une réduction suffisante de la pression car l'acide acrylique cristallise dans le condenseur de tête lorsque la pression règnant dans la colonne est abaissée à une valeur inférieure à 5 mm Hg. Suivant un trait fondamental de l'invention, on maintient dans le pied de la colonne une quantité d'acide acrylique au moins suffisante pour pouvoir effectuer la distillation dans les conditions de températures recherchées. La quantité d'acide acrylique à maintenir dans la solution peut se situer entre 1 et 20 %, de préférence 2 à 10 % pour la gamme de températures préférée citée plus haut. Comme dans le stade précédent, le temps de séjour revêt une certaine importance; il est également d'autant plus court que la température est plus élevée; pour une température de 120° à 150°C, il est comprise entre 1 heure et 30 secondes. La pression est ajustée en fonction de la température et de la teneur en acide acrylique choisies dans chaque cas particulier et sa détermination est à la portée de l'homme de l'art. Une gamme de pression comprise entre 5 et 30 mm Hg convient généralement pour l'ensemble des cas.

Le liquide sortant au pied de la colonne, composé de phosphate de tri-n-butyle et d'une certaine quantité d'acide acrylique, est avantageusement recyclé au stade de vaporisation qui constitue le premier stade du procédé selon la présente invention. On conçoit aisément l'intérêt de ce recyclage grâce auquel on peut recueillir, sur l'ensemble du procédé, la totalité de l'acide acrylique et du phosphate de tri-n-butyle contenus dans la solution de départ. Un mode de mise en oeuvre du procédé peut d'ailleurs constituer à régler le fonctionnement de la colonne de distillation de telle sorte que le liquide sortant au pied de la colonne et recyclé au premier stade ait une concentration du même ordre que celle de la solution de départ. Toutefois ce mode de mise en oeuvre n'est pas impératif et l'on peut parfaitement travailler avec des solutions initiales et de recyclage ayant des concentrations différentes.

Bien entendu, on ne sort pas du cadre de l'invention en ne recyclant pas au premier stade le liquide soutiré du pied de la colonne de distillation, et en utilisant ce liquide dans une autre application.

Le procédé selon la présente invention peut comporter un certain nombre de variantes applicables séparément ou simultanément.

Selon une première variante, si la solution de départ contient, à côté d'acide acrylique, d'autres acides organiques légers, notamment de l'acide acétique, on procède dans un stade complémentaire, à la distillation de cet acide. Cette séparation s'effectue préalablement à la distillation de l'acide acrylique. Les conditions de température et de pression sont à rapprocher de celles de la distillation de l'acide acrylique c'est-à-dire qu'il est nécessaire d'appliquer une température en pied de colonne qui n'entraine pas la dégradation du phosphate de tributyle et de ne pas abaisser la pression à un point tel qu'il en résulte une congélation de l'acide acétique. Une température de l'ordre de 120—150°C et une pression de 30 à 100 mm de mercure sont des conditions de travail favorables.

Selon une seconde variante, particulièrement intéressante lorsqu'on applique le procédé à des solutions relativement concentrées en acide acrylique, on introduit tout ou partie de la solution à traiter, en aval du dispositif de vaporisation, de préférence dans la colonne de distillation elle-même, à un niveau où la concentration en acide acrylique est sensiblement identique à celle de la solution d'alimentation. Une partie de l'acide acrylique distille et le liquide restant dans le pied de la colonne est envoyé dans le dispositif de vaporisation. En d'autres termes, l'ensemble du cycle opératoire reste par ailleurs inchangé.

On peut aussi introduire dans le circuit des fluides, par exemple dans le condenseur de tête de la ou des colonnes de distillation, de inhibiteurs de polymérisation de type connu, comme l'hydroquinone, l'éther méthylique de l'hydroquinone ou autres.

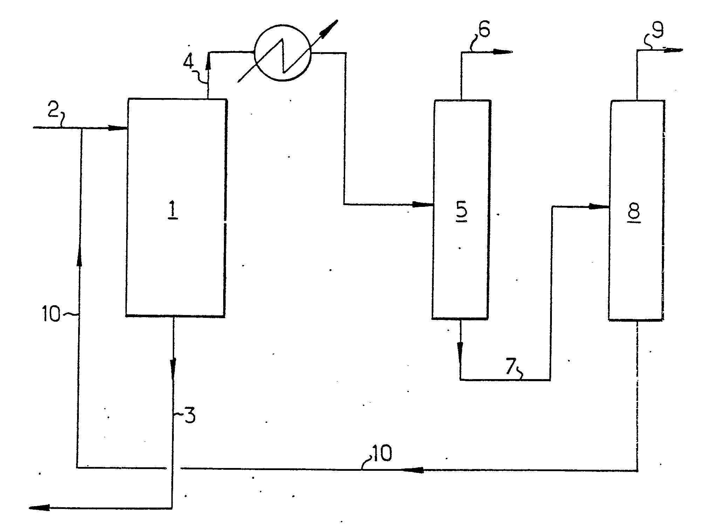

L'invention sera mieux comprise par la description ci-après d'un mode de mise en oeuvre du procédé, où l'on se réfère au schéma de principe de la figure 1 annexée. Le dispositif de vaporisation 1, par exemple un évaporateur à film mince, est alimenté en solution à traiter par la conduite 2. Au bas du dispositif 1, le phosphate de tri-n-butyle débarrassé de l'acide acrylique et de tout autre acide éventuel est soutiré et envoyé par la ligne 3 au stade d'absorption ou d'extraction d'acide acrylique, en amont du procédé selon l'invention. La phase vapeur contenant l'acide acrylique, éventuellement des acides légers comme l'acide acétique et une certaine quantité de phosphate de tri-n-butyle sort de l'appareil 1 par la conduite 4, puis après condensation partielle ou totale, est introduite dans la colonne 5 où l'acide acétique passe en tête et est recueilli par la canalisation 6. La phase liquide restante s'écoule par la conduite 7 et alimente la colonne de distillation 8 où l'acide acrylique s'échappe par 9 tandis que le liquide soutiré au pied de la colonne est recyclé par la conduite 10 à l'alimentation du dispositif 1. La colonne 5 est facultative et est supprimée si la solution d'alimentation ne contient pas d'acides autres que l'acide acrylique. Le procédé selon la présente invention peut être réalisé en continu ou en discontinu.

L'exemple ci-après illustre l'invention sans toutefois la limiter.

### Exemple

Dans cet exemple, on se réfère au schéma de la figure 1 annexée. L'évaporateur à film mince (1) est alimenté par la tubulure (2) avec 108,78 parties en poids d'un mélange contenant 6,52 % poids d'acide acrylique 93,30 % de tributylphosphate, 0,01 % d'eau et 0,17 % d'acide acétique. Ce mélange provient pour 82,66 parties d'un système d'absorption d'acide acrylique par le phosphate de tri-n-butyle et pour 26,12 parties d'un recyclage par la conduite 10.

L'évaporateur (1) est maintenu, au moyen d'un système approprié, sous une pression de 5 mm de mercure et à une température de 128°C. Il se produit une séparation en une fraction liquide et une fraction vapeur.

La fraction liquide soutirée par (3), en bas de l'appareil, représente 76,77 parties contenant 0,1 % poids d'acide acrylique et 99,9 % de tributylphosphate. Ce liquide, après un refroidissement convenable est renvoyé à l'appareil d'extraction d'acide acrylique.

La fraction vapeur, sortant en (4), après condensation partielle ou totale est envoyée dans la colonne à distiller (5). Elle représente 32,01 parties en poids contenant 21,90 % poids d'acide acrylique, 77,46 % de tributylphosphate, 0,59 % d'acide acétique et 0,03 % d'eau. Destillationseinheit abgetrennte und in der flüssigen Phase konzentrierte Tri-n-butylphosphat zur Verdampfungsphase zurückführt.

6. Verfahren nach einem der Ansprüche 1 bis 5, dadurch gekennzeichnet, daß man die gesamte oder einen Teil der zu behandelnden Lösung nach der Verdampfungsvorrichtung und vorzugsweise in die Destillationskolonne einführt.

### Claims

1. Process for obtaining pure acrylic acid, consisting of absorbing the reaction gas resulting from the oxidation of propylene and/or of acrolein by means of an absorption agent and isolating, from this solution, firstly the pure acrylic acid and secondly the absorption agent in a sufficient degree of purity to permit its recycling, characterised in that the absorption agent is tri-n-butyl phosphate and the separation of the tri-n-butyl phosphate from the acrylic acid is carried out by a combination of heat treatments which avoids both the decomposition of tri-n-butyl phosphate and the crystallisation of acrylic acid in the apparatus, the said combination consisting of:

1 — producing, in a first apparatus, a vapour phase which essentially contains all the acrylic acid and a part of the tri-n-butyl phosphate, by heating the solution at between 110 and 150°C under 1 to 33 mm or mercury and with a residence time of less than 30 minutes, so as to avoid the degradation of the tri-n-butyl phosphate, and

2 — continuously treating the 2 phases thus produced, as follows:

on the one hand, recycling, as absorption agent, the tri-n-butyl phosphate concentrated in the liquid phase and, on the other hand, removing the gaseous mixture of acrylic acid and tri-n-butyl phosphate to a distillation unit where the mixture, which may be completely or partly condensed, is treated so that the product at the bottom of the column contains 1 to 20% of acrylic acid, the temperature at the foot of the column is between 120 and 150°C under 5 to 30 mm of mercury, and the residence time does not exceed 1 hour, so that the degradation of tri-n-butyl phosphate and the crystallisation of acrylic acid at the top of the column are avoided.

2. Process according to Claim 1, characterised in that in the vaporisation stage the residence time is between 30 minutes and 5 seconds.

3. Process according to one of Claims 1 and 2, characterised in that in the distillation stage the residence time is between 1 hour and 30 seconds.

4. Process according to one of Claims 1 to 3, characterised in that the product at the bottom of the column contains from 2 to 10% by weight of acrylic acid.

5. Process according to one of Claims 1 to 4, characterised in that the tri-n-butyl phosphate separated off in the distillation unit and concentrated in the liquid phase is recycled to the vaporisation stage.

6. Process according to one of Claims 1 to 5, characterised in that all or part of the solution to be treated is introduced downstream of the vaporisation device and preferably into the distillation column.

La colonne (5) fonctionne sous 35 mm de mercure en tête et 42 mm en pied correspondant à des températures respectives de 40°C et 120°C. La présence du phosphate de tributyle permet la séparation en tête par (6) de 0,19 parties en poids d'un effluent contenant 89,47 % poids d'acide acétique avec 5,26 % d'acide acrylique et 5,26 % d'eau. Le liquide sortant du pied de la colonne en (7) représente 31,82 parties en poids contenant 77,94 % de tributylphosphate, 22,00 % d'acide acrylique et 0,06 % d'acide acétique.

Ce produit est envoyé dans la colonne (8) fonctionnant sous 10 mm de mercure en tête et 12 mm en pied. Les températures sont respectivement de 40 et 130°C.

La fraction de tête (9) de 5,7 parties en poids correspond à la production d'acide acrylique à une pureté de 99,65 %. L'effluent s'écoulant par 10, composé de 26,12 parties d'un liquide contenant 94,95 % de phosphate de tributyle et 5,05 % d'acide acrylique, est recyclé à l'alimentation de l'appareil 1.

## Revendications

1. Procédé d'obtention d'acide acrylique pur consistant à absorber le gaz réactionnel issu de l'oxydation du propylène et/ou de l'acroléïne par un agent d'absorption et à séparer de cette solution d'une part l'acide acrylique pur et d'autre part l'agent d'absorption à un degré de pureté suffisant pour son recyclage, caractérisé en ce que:

l'agent d'absorption est le phosphate de tri-n-butyle et la séparation du phosphate de tri-n-butyle et de l'acide acrylique est réalisée par une combinaison de traitements thermiques évitant à la fois la décomposition du phosphate de tri-n-butyle et la cristallisation de l'acide acrylique dans les appareils, ladite combinaison consistant:

1 — à créer dans un premier appareil une phase vapeur comprenant sensiblement tout l'acide acrylique et une partie du phosphate de tri-n-butyle par chauffage de la solution entre 110 et 150°C sous 1 à 30 mm de mercure et avec un temps de séjour inférieur à 30 minutes de manière à éviter la dégradation du phosphate de tri-n-butyle.

2 — à traiter en continu les 2 phases ainsi créées comme suit:

d'une part, recycler comme agent d'absorption le phosphate de tri-n-butyle concentré dans la phase liquide et d'autre part, évacuer le mélange gazeux d'acide acrylique et de phosphate de tri-n-butyle vers une unité de distillation où le mélange éventuellement condensé en tout ou en partie est traité de manière à ce que le produit en bas de colonne contienne de 1 à 20 % d'acide acrylique, à ce que la température en pied de colonne se situe entre 120 et 150°C sous 5 à 30 mm de mercure et que le temps de séjour ne soit pas supérieure à 1 heure de telle façon que la dégradation du phosphate de tri-n-butyle et la cristallisation de l'acide acrylique en tête de colonne soit évitée.

2. Procédé selon la revendication 1, caractérisé en ce que dans le stade de la vaporisation, le temps de séjour est comprise entre 30 minutes et 5 secondes.

3. Procédé selon l'une des revendications 1 et 2, caractérisé en ce que dans le stade de la distillation le temps de séjour est comprise entre 1 heure et 30 secondes.

4. Procédé selon l'une des revendications 1 à 3, caractérisé en ce que le produit en bas de colonne contient de 2 à 10 % en poids d'acide acrylique.

5. Procédé selon l'une des revendications 1 à 4 caractérisé en ce que l'on recycle au stade de la vaporisation le phosphate de tri-n-butyle séparé dans l'unité de distillation et concentré dans la phase liquide.

6. Procédé selon l'une des revendications 1 à 5, caractérisé en ce que l'on introduit tout ou partie de la solution à traiter en aval du dispositif de vaporisation et de préférence dans la colonne de distillation.

## Patentansprüche

1. Verfahren zur Herstellung von reiner Acrylsäure, bei dem das aus der Oxydation von Propylen und/oder Acrolein stammende Reaktionsgas durch ein Absorptionsmittel absorbiert wird, und die reine Acrylsäure einerseits und das Absorptionsmittel mit einer für seine Rückführung ausreichenden Reinheit andererseits von dieser Lösung abgetrennt werden, dadurch gekennzeichnet, daß das Absorptionsmittel Tri-n-butylphosphat ist, und die Trennung des Tri-n-butylphosphats von der Acrylsäure durch eine Kombination von Wärmebehandlungen erfolgt, die sowohl die Zersetzung des Tri-n-butylphosphats und die Kristallisation de Acrylsaüre in den Apparaten vermeidet und darin besteht, daß

1 — in einem ersten Apparat durch Erhitzen der Lösung auf 110 bis 150°C unter einem Druck von 1 bis 30 mm Hg und bei einer Verweilzeit von weniger als 30 Minuten, um den Abbau des Tri-n-butylphosphats zu vermeide eine Dampfphase gebildet wird, die im wesentlichen. die gesamte Acrylsäure und einen Teil des Tri-n-Butylphosphats enthält und daß

2 — die beiden so gebildeten Phasen wie folgt kontinuierlich behandelt werden:

Einerseits Rückführung des konzentrierten Tri-n-butylphosphats in die flüssige Phase als Absorptionsmittel und andererseits Überführung der gasförmigen Mischung aus Acrylsäure und Tri-n-butylphosphat in eine Destillationseinheit, wo das gegebenenfalls ganz oder teilweise kondensierte Gemisch so behandelt wird, daß das Sumpfprodukt der Kolonne 1 bis 20 % Acrylsäure enthält, daß die Temperatur im Sumpf der Kolonne bei 5 bis 30 mm Hg zwischen 120 und 150°C liegt, und daß die Verweilzeit nicht über eine Stunde dauert, damit die Zersetzung des Tri-n-butylphosphats und die Kristallisation der Acrylsäure am Kopf der Kolonne vermieden wird.

2. Verfahren nach Anspruch 1, dadurch gekennzeichnet, daß im Verdampfungsstadium die Verweilzeit zwischen 30 Minuten und 5 Sekunden beträgt.

3. Verfahren nach Anspruch 1 oder 2, dadurch gekennzeichnet, daß im Destillationsstadium die Verweilzeit zwischen einer Stunde und 30 Sekunden beträgt.

4. Verfahren nach einem der Ansprüche 1 bis 3, dadurch gekennzeichnet, daß das Sumpfprodukt der Kolonne 2 bis 10 Gew.% Acrylsäure enthält.

5. Verfahren nach einem der Ansprüche 1 bis 4, dadurch gekennzeichnet, daß man das in der

colonne de distillation de telle sorte que le liquide sortant au pied de la colonne et recyclé au premier stade ait une concentration du même ordre que celle de la solution de départ. Toutefois ce mode de mise en oeuvre n'est pas impératif et l'on peut parfaitement travailler avec des solutions initiales et de recyclage ayant des concentrations différentes.

Bien entendu, on ne sort pas du cadre de l'invention en ne recyclant pas au premier stade le liquide soutiré du pied de la colonne de distillation, et en utilisant ce liquide dans une autre application.

Le procédé selon la présente invention peut comporter un certain nombre de variantes applicables séparément ou simultanément.

Selon une première variante, si la solution de départ contient, à côté d'acide acrylique, d'autres acides organiques légers, notamment de l'acide acétique, on procède dans un stade complémentaire, à la distillation de cet acide. Cette séparation s'effectue préalablement à la distillation de l'acide acrylique. Les conditions de température et de pression sont à rapprocher de celles de la distillation de l'acide acrylique c'est-à-dire qu'il est nécessaire d'appliquer une température en pied de colonne qui n'entraine pas la dégradation du phosphate de tributyle et de ne pas abaisser la pression à un point tel qu'il en résulte une congélation de l'acide acétique. Une température de l'ordre de 120—150°C et une pression de 30 à 100 mm de mercure sont des conditions de travail favorables.

Selon une seconde variante, particulièrement intéressante lorsqu'on applique le procédé à des solutions relativement concentrées en acide acrylique, on introduit tout ou partie de la solution à traiter, en aval du dispositif de vaporisation, de préférence dans la colonne de distillation elle-même, à un niveau où la concentration en acide acrylique est sensiblement identique à celle de la solution d'alimentation. Une partie de l'acide acrylique distille et le liquide restant dans le pied de la colonne est envoyé dans le dispositif de vaporisation. En d'autres termes, l'ensemble du cycle opératoire reste par ailleurs inchangé.

On peut aussi introduire dans le circuit des fluides, par exemple dans le condenseur de tête de la ou des colonnes de distillation, de inhibiteurs de polymérisation de type connu, comme l'hydroquinone, l'éther méthylique de l'hydroquinone ou autres.

L'invention sera mieux comprise par la description ci-après d'un mode de mise en oeuvre du procédé, où l'on se réfère au schéma de principe de la figure 1 annexée. Le dispositif de vaporisation 1, par exemple un évaporateur à film mince, est alimenté en solution à traiter par la conduite 2. Au bas du dispositif 1, le phosphate de tri-n-butyle débarrassé de l'acide acrylique et de tout autre acide éventuel est soutiré et envoyé par la ligne 3 au stade d'absorption ou d'extraction d'acide acrylique, en amont du procédé selon l'invention. La phase vapeur contenant l'acide acrylique, éventuellement des acides légers comme l'acide acétique et une certaine quantité de phosphate de tri-n-butyle sort de l'appareil 1 par la conduite 4, puis après condensation partielle ou totale, est introduite dans la colonne 5 où l'acide acétique passe en tête et est recueilli par la canalisation 6. La phase liquide restante s'écoule par la conduite 7 et alimente la colonne de distillation 8 où l'acide acrylique s'échappe par 9 tandis que le liquide soutiré au pied de la colonne est recyclé par la conduite 10 à l'alimentation du dispositif 1. La colonne 5 est facultative et est supprimée si la solution d'alimentation ne contient pas d'acides autres que l'acide acrylique. Le procédé selon la présente invention peut être réalisé en continu ou en discontinu.

L'exemple ci-après illustre l'invention sans toutefois la limiter.

### Exemple

Dans cet exemple, on se réfère au schéma de la figure 1 annexée. L'évaporateur à film mince (1) est alimenté par la tubulure (2) avec 108,78 parties en poids d'un mélange contenant 6,52 % poids d'acide acrylique 93,30 % de tributylphosphate, 0,01 % d'eau et 0,17 % d'acide acétique. Ce mélange provient pour 82,66 parties d'un système d'absorption d'acide acrylique par le phosphate de tri-n-butyle et pour 26,12 parties d'un recyclage par la conduite 10.

L'évaporateur (1) est maintenu, au moyen d'un système approprié, sous une pression de 5 mm de mercure et à une température de 128°C. Il se produit une séparation en une fraction liquide et une fraction vapeur.

La fraction liquide soutirée par (3), en bas de l'appareil, représente 76,77 parties contenant 0,1 % poids d'acide acrylique et 99,9 % de tributylphosphate. Ce liquide, après un refroidissement convenable est renvoyé à l'appareil d'extraction d'acide acrylique.

La fraction vapeur, sortant en (4), après condensation partielle ou totale est envoyée dans la colonne à distiller (5). Elle représente 32,01 parties en poids contenant 21,90 % poids d'acide acrylique, 77,46 % de tributylphosphate, 0,59 % d'acide acétique et 0,03 % d'eau. Destillationseinheit abgetrennte und in der flüssigen Phase konzentrierte Tri-n-butylphosphat zur Verdampfungsphase zurückführt.

6. Verfahren nach einem der Ansprüche 1 bis 5, dadurch gekennzeichnet, daß man die gesamte oder einen Teil der zu behandelnden Lösung nach der Verdampfungsvorrichtung und vorzugsweise in die Destillationskolonne einführt.

### Claims

1. Process for obtaining pure acrylic acid, consisting of absorbing the reaction gas resulting from the oxidation of propylene and/or of acrolein by means of an absorption agent and isolating, from this solution, firstly the pure acrylic acid and secondly the absorption agent in a sufficient degree of purity to permit its recycling, characterised in that the absorption agent is tri-n-butyl phosphate and the separation of the tri-n-butyl phosphate from the acrylic acid is carried out by a combination of heat treatments which avoids both the decomposition of tri-n-butyl phosphate and the crystallisation of acrylic acid in the apparatus, the said combination consisting of:

1 — producing, in a first apparatus, a vapour phase which essentially contains all the acrylic acid and a part of the tri-n-butyl phosphate, by heating the solution at between 110 and 150°C under 1 to 33 mm or mercury and with a residence time of less than 30 minutes, so as to avoid the degradation of the tri-n-butyl phosphate, and

2 — continuously treating the 2 phases thus produced, as follows:

on the one hand, recycling, as absorption agent, the tri-n-butyl phosphate concentrated in the liquid phase and, on the other hand, removing the gaseous mixture of acrylic acid and tri-n-butyl phosphate to a distillation unit where the mixture, which may be completely or partly condensed, is treated so that the product at the bottom of the column contains 1 to 20% of acrylic acid, the temperature at the foot of the column is between 120 and 150°C under 5 to 30 mm of mercury, and the residence time does not exceed 1 hour, so that the degradation of tri-n-butyl phosphate and the crystallisation of acrylic acid at the top of the column are avoided.

2. Process according to Claim 1, characterised in that in the vaporisation stage the residence time is between 30 minutes and 5 seconds.

3. Process according to one of Claims 1 and 2, characterised in that in the distillation stage the residence time is between 1 hour and 30 seconds.

4. Process according to one of Claims 1 to 3, characterised in that the product at the bottom of the column contains from 2 to 10% by weight of acrylic acid.

5. Process according to one of Claims 1 to 4, characterised in that the tri-n-butyl phosphate separated off in the distillation unit and concentrated in the liquid phase is recycled to the vaporisation stage.

6. Process according to one of Claims 1 to 5, characterised in that all or part of the solution to be treated is introduced downstream of the vaporisation device and preferably into the distillation column.

La colonne (5) fonctionne sous 35 mm de mercure en tête et 42 mm en pied correspondant à des températures respectives de 40°C et 120°C. La présence du phosphate de tributyle permet la séparation en tête par (6) de 0,19 parties en poids d'un effluent contenant 89,47 % poids d'acide acétique avec 5,26 % d'acide acrylique et 5,26 % d'eau. Le liquide sortant du pied de la colonne en (7) représente 31,82 parties en poids contenant 77,94 % de tributylphosphate, 22,00 % d'acide acrylique et 0,06 % d'acide acétique.

Ce produit est envoyé dans la colonne (8) fonctionnant sous 10 mm de mercure en tête et 12 mm en pied. Les températures sont respectivement de 40 et 130°C.

La fraction de tête (9) de 5,7 parties en poids correspond à la production d'acide acrylique à une pureté de 99,65 %. L'effluent s'écoulant par 10, composé de 26,12 parties d'un liquide contenant 94,95 % de phosphate de tributyle et 5,05 % d'acide acrylique, est recyclé à l'alimentation de l'appareil 1.

**Revendications**

1. Procédé d'obtention d'acide acrylique pur consistant à absorber le gaz réactionnel issu de l'oxydation du propylène et/ou de l'acroléïne par un agent d'absorption et à séparer de cette solution d'une part l'acide acrylique pur et d'autre part l'agent d'absorption à un degré de pureté suffisant pour son recyclage, caractérisé en ce que:

l'agent d'absorption est le phosphate de tri-n-butyle et la séparation du phosphate de tri-n-butyle et de l'acide acrylique est réalisée par une combinaison de traitements thermiques évitant à la fois la décomposition du phosphate de tri-n-butyle et la cristallisation de l'acide acrylique dans les appareils, ladite combinaison consistant:

1 — à créer dans un premier appareil une phase vapeur comprenant sensiblement tout l'acide acrylique et une partie du phosphate de tri-n-butyle par chauffage de la solution entre 110 et 150°C sous 1 à 30 mm de mercure et avec un temps de séjour inférieur à 30 minutes de manière à éviter la dégradation du phosphate de tri-n-butyle.

2 — à traiter en continu les 2 phases ainsi créées comme suit:

d'une part, recycler comme agent d'absorption le phosphate de tri-n-butyle concentré dans la phase liquide et d'autre part, évacuer le mélange gazeux d'acide acrylique et de phosphate de tri-n-butyle vers une unité de distillation où le mélange éventuellement condensé en tout ou en partie est traité de manière à ce que le produit en bas de colonne contienne de 1 à 20 % d'acide acrylique, à ce que la température en pied de colonne se situe entre 120 et 150°C sous 5 à 30 mm de mercure et que le temps de séjour ne soit pas supérieure à 1 heure de telle façon que la dégradation du phosphate de tri-n-butyle et la cristallisation de l'acide acrylique en tête de colonne soit évitée.

2. Procédé selon la revendication 1, caractérisé en ce que dans le stade dela vaporisation, le temps de séjour est comprise entre 30 minutes et 5 secondes.